# DEMANDE DE BREVET EUROPEEN

(11) **EP 0 596 772 A1**
(43) Date de publication de la demande: **11.05.1994**
(21) Numéro de dépôt: 93402620.4
(22) Date de dépôt: 26.10.1993
(51) Int. Cl.: A61L 9/04, A61L 9/01, C08K 5/09, C08K 5/10, C08L 77/12

(54) **Composition contenant de l'acide undécylènique ou ses dérivés**

(30) Priorité: 06.11.1992 FR 9213369
(71) Demandeur: ELF ATOCHEM S.A., F-92800 Puteaux (FR)
(72) Inventeur: Caupin, Henri-Jean, F-78000 Versailles (FR); Leroux, Rolland, F-92370 Chaville (FR)
(74) Mandataire: Hirsch, Marc-Roger

(57) **Abrégé**

La présente invention a pour objet une composition stable comprenant, en poids par rapport au poids de la composition: (a) de 90 à 5% en poids d'un polymère polyéther-esteramide séquencé et (b) de 10 à 95% en poids d'acide undécylénique ou ses dérivés, calculé sur la base du poids en acide undécylénique. La présente invention a aussi pour objet le procédé de préparation de ces compositions.

La présente composition a une action désodorisante.

## Description

La présente invention a pour objet une nouvelle composition, plus particulièrement une nouvelle résine polymère chargée en acide undécylénique, ainsi qu'un procédé de préparation de celle-ci.

L'acide undécylénique, ou ses dérivés tels que les esters ou les sels de métaux en particulier alcalins ou alcalino-terreux est connu pour ses nombreuses propriétés.

Plusieurs documents décrivent l'action pédiculicide de l'acide undécylénique ou ses dérivés, notamment esters d'alkyles inférieurs.

Plusieurs documents décrivent l'action fongicide de l'acide undécylénique.

Plusieurs documents décrivent l'action désodorisante de cet acide, en ce sens que cet acide en C₁₁ permet de débarrasser l'atmosphère des odeurs viciées.

En fait, comme dans le cas de l'action désodorisante, par exemple, il serait avantageux de fournir un support pour cet acide ou ses dérivés, afin d'obtenir des "sticks déodorants", faciles d'emploi.

FR-A-2 579 983 décrit une résine polymère à base de polyétheresteramide (PEEA), en tant que support pour des parfums, ladite résine assurant la libération de ceux-ci. Ce brevet a pour objet la libération d'un parfum, soit une action odorisante par un mécanisme de diffusion avec un effet de masque. Il n'est fait aucune mention d'une action désodorisante ou d'une quelconque destruction des mauvaises odeurs.

Ainsi, la présente invention a pour objet une composition stable comprenant, sur la base du poids total de la composition:
(a) de 90 à 5% en poids d'un polymère polyétheresteramide séquencé de formule: 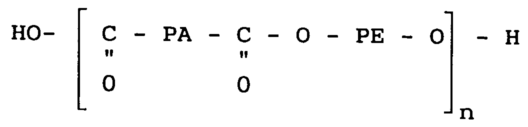 dans laquelle PA représente le segment polyamide et PE représente le segment polyéther, n est un entier représentant la répartition des unités récurrentes; et
(b) 10 à 95% en poids, d'acide undécylénique ou ses dérivés, calculé sur la base du poids en acide undécylénique.

Ce polymère PEEA est obtenu par tout procédé approprié; en particulier la résine polyéther-ester-amide est obtenue par réaction à l'état fondu entre un polyamide dicarboxylique à fonctions carboxyliques terminales d'un poids moléculaire moyen compris entre 300 et 15000 et un polyoxyalkylène glycol aliphatique linéaire ou ramifié à fonctions hydroxyles terminales et d'un poids moléculaire moyen compris entre 200 et 6000, sous vide poussé à des températures comprises entre 100 et 400°C en présence d'un catalyseur qui est par exemple un tétraalkyl-orthotitanate de formule générale Ti(OR)₄; R étant un radical hydrocarboné aliphatique linéaire ou ramifié dont le nombre d'atomes de carbone est compris entre 1 et 24 et dont la teneur en poids par rapport au mélange réactionnel est comprise entre 0,01 et 5%.

De tels polymères sont disponibles dans le commerce, par exemple sous la dénomination PEBAX®, de Elf Atochem.

Par dérivés de l'acide undécylénique, on entend les dérivés tant hydrosolubles que liposolubles. De tels dérivés préférés sont les esters d'alkyle en C₁ à C₆, avantageusement C₁ à C₃, comme les esters méthylique, éthylique, isopropylique.

La composition contient de préférence 50 à 10% de polymère polyétheresteramide séquencé et 50 à 90% d'acide undécylénique ou ses dérivés. Ainsi, on peut noter la capacité remarquable d'absorption des PEEA vis-à-vis des dérivés de l'acide undécylénique.

La composition peut être utilisée telle quelle, c'est-à-dire être façonnée en diverses formes, fonctionnelles ou décoratives. Elle peut être façonnée aisément par extrusion ou par moulage selon les techniques usuelles pour la transformation de matériaux polymères thermoplastiques. La composition selon l'invention peut aussi contenir d'autres additifs, par exemple les solvants de l'acide, ces additifs étant présents en des quantités variables, généralement jusqu'à 50% en poids de la composition finale ainsi obtenue.

La composition selon l'invention peut, par ailleurs, être utilisée pour la préparation de concentrés qui, par exemple, sous forme de granulés ou d'extrudats, seront incorporés dans d'autres polymères thermoplastiques, selon la méthode dite du "Master Batch".

Cette méthode du Master Batch est décrite, par exemple, dans Rubber & Plastics, 423283 (1961). Ces polymères thermoplastiques peuvent être, par exemple, des (co)polyoléfines, l'EVA, le PVC et autres.

Ainsi, selon un mode de réalisation, la présente invention a pour objet les articles façonnés obtenus à partir de la présente composition.

Une application particulière de la présente invention est la fabrication de semelle intérieure de chaussure comprenant une composition selon l'invention. Cette semelle peut être fixe dans la chaussure ou amovible et glissée dans la chaussure.

Selon un autre mode de réalisation, la présente invention a pour objet un mélange-maître ou "Master Batch" obtenu à partir de la présente composition.

Toute technique d'imprégnation du PEEA et appropriée. Cependant, il a été trouvé qu'un procédé particulier est particulièrement avantageux. Ainsi, la présente invention a aussi pour objet un procédé de préparation de la composition.

Selon la présente invention, ce procédé est caractérisé en ce que l'acide undécylénique et/ou ses dérivés est incorporé dans un alcool selon un rapport volumique calculé par rapport à la forme acide compris entre 1/9 et 9/1, et imprégnation de polyétheresteramide avec le mélange résultant.

Selon une variante du procédé selon l'invention, l'alcool est ensuite partiellement ou totalement éliminé. Ceci peut être effectué par exemple par évaporation, ce qui conduit à un produit final. Ce produit final ainsi obtenu fait aussi partie de la présente invention.

L'alcool utilisé est de préférence un alcool en C₁ à C₆ saturé, avantageusement le méthanol.

Les proportions d'acide et d'alcool peuvent varier dans une large mesure, cependant on préfère un rapport volumique compris entre 1/2 et 2/1.

La présente invention est maintenant décrite plus en détail par les exemples suivants; dans ceux-ci, ainsi que dans la description et les revendications, les pourcentages en poids sont calculés par rapport à la composition finale, et par rapport à la forme acide en ce qui concerne les dérivés, sauf mention du contraire.

### Exemples 1 et 2.

### Préparation de la composition

On utilise deux PEEA, dont les dénominations sont PEBAX® 3533 et 2533 respectivement (Elf Atochem), mis sous forme de billes d'un diamètre d'environ 3 mm.

### Caractérisation des échantillons

Les échantillons de PEBAX ont été caractérisés; les résultats sont résumés dans le tableau ci-après.

| Référence | 3533 | 2533 |
|---|---|---|
| Perte de poids à 40° | 0,22 % | 0,24 % |
| pendant 96 heures | | |
| Point de fluidisation | 170°C | |
| Point de figeage | 120 - 130°C | |
| Densité apparente | 0,588 | 0,564 |
| Variation pondérale (24h à 105°C) | | |
| Augmentation de poids | 1,12 % | 1,41 % |

### Mesure des capacités d'absorption de liquides

Les liquides étudiés ont été les suivants:
- eau déminéralisée,
- éthanol,
- méthanol,
- Undécylénate de méthyle pur,
- Mélange de 50% en volume d'undécylénate de méthyle et de méthanol,
- Mélange de 50% en volume d'acide undécylénique et de méthanol.

Les capacités d'absorption sont déterminées de la façon suivante:

On prend 20 g de billes de PEBAX qui sont immergées dans 300 ml de liquide, porté à 450 ml pour le dernier mélange (Méthanol + Acide undécylénique). Pour l'eau, on utilise 100 g de billes.

La capacité d'absorption a été mesurée après 24 heures à la température du laboratoire. On a filtré les billes puis on les a séchées et essorées sur papier filtre jusqu'à obtention de billettes d'apparence sèches susceptibles de s'écouler sans adhérence sur une surface de verre. On a mesuré le poids et le volume apparent des billettes gonflées.

Les résultats sont compilés dans le tableau suivant:

| Référence | 3533 | | 2533 | |
|---|---|---|---|---|
| Liquide absorbé | Poids | Volume | Poids | Volume |
| Eau (Po = 100 g) | 102,29 | 170 | 103,09 | 173 |
| Méthanol (Po = 20 g) | 30,12 | 55 | 32,23 | 59 |
| (Une partie est soluble dans le méthanol: | 0,62 | | 0,51) | |
| Und Met. pur. | 33,25 | 57 | 37,98 | 64 |
| Und Met 50%V/MetOH 50%V. | 78,69 | 140 | 152,50 | 254 |
| Ac. Und 50%V/MetOH 50%V. | 137,77 | 220 | 251 | 360 |

Les résultats sont présentés sous forme de ratios significatifs, c'est-à-dire en définissant:
- un taux d'absorption en % pondéraux (T = 100x(Pf-Po)/Po),
- une densité apparente des billettes finales (d = Pf/Vf).

où
Pf est le poids final
Po est le poids initial
Vf est le volume final.

Le tableau ci-dessous présente les taux d'absorption et poids spécifique des billettes.

| Référence | 3533 | | 2533 | |
|---|---|---|---|---|
| Liquide absorbé | Taux % | d. g/l | Taux % | d. g/l. |
| Eau (Po = 100 g) | 2,29 | 0,302 | 3,09 | 0,596 |
| Méthanol (Po = 20 g) | 50,60 | 0,547 | 61,15 | 0,546 |
| Und Met. pur. | 66,25 | 0,583 | 89,90 | 0,593 |
| Und Met 50%V/MetOH 50%V. | 293,45 | 0,562 | 662,50 | 0,600 |
| Ac. Und 50%V/MetOH 50%V. | 588,85 | 0,626 | 1155,00 | 0,697 |

Les billettes ayant absorbé du méthanol et de l'undécylénate de méthyle conservent une certaine rigidité.

Quant aux mélanges de MetOH et C11, les billettes prennent l'aspect d'un gel consistant.

Le méthanol a tendance à s'évaporer dans les billettes laissées à l'atmosphère.

La fusion des billettes est modifiée par la présence des produits absorbés. L'échantillon 3533 imprégné d'undécylénate de méthyle devient fluide à 130-140°C et présente un point de figeage à 80°C. On observe une perte en poids de 5,73%.

Le même échantillon imprégné d'acide Undécylénique et de méthanol se comporte comme suite. On observe une fluidisation totale dès 45-50°C, le liquide est homogène et très peu visqueux, puis on atteint le point d'évaporation du méthanol à 65°C, le liquide reste toujours fluide jusqu'à la disparition totale du méthanol. Le figeage se fait alors, mais, si l'on continue de chauffer, il reste fluide et le figeage en refroidissement s'accomplit vers 70°C.

On observe une perte en poids de 44,30%, soit la part correspondant au méthanol.

La composition de la billette saturée était la suivante:
- PEBAX : 7,96%
- Acide undécylénique : 47,80%
- MetOH : 44,17%

Le mélange figé présente la composition suivante:
- PEBAX : 14,30%
- Acide undécylénique : 85,72%

L'éprouvette obtenue présente une certaine résistance mécanique et une bonne consistance.

### Mesure de l'efficacité des PEBAX "Charges" en désodorisation Mode opératoire

L'approche a été réalisée par mesure des seuils de perception des odeurs testées. le dispositif expérimental retenu revient à tester deux configurations de situation:
- en phase dynamique
   Cette approche simule un conditionnement d'air sans recyclage.
   Dans cette hypothèse, les odeurs de base (mauvaises odeurs) sont générées par un dispositif séparé (passage forcé dans une colonne) et un flux calibré de cette odeur de base (q) est envoyé dans une autre colonne remplie de modules de Pebax additivé en C11, lequel flux est ensuite dilué dans un débit (Q) d'air pur.
   Ce dispositif permet par un jeu de vannes et dérivation approprié, et en faisant varier le rapport (q/Q) de définir le seuil de perception de:
   * l'odeur de base (niveau N maximum)
   * l'odeur du Pebax (niveau à zéro ou à N minimum),
   * l'odeur du flux de base passé sur Pebax (niveau N compris entre les deux précédents).

   La situation de N par rapport à N maximum et N minimum permet de déterminer l'efficacité du Pebax additivé pour l'usage préfiguré.
- en phase statique
   Cette approche simule un état d'équilibre en enceinte close.
   Dans cette hypothèse, les odeurs de base (mauvaises odeurs) sont générées au sein d'une enceinte contenant des modules de Pebax additivé en C11. Un débit d'air pur (q) est envoyé dans cette enceinte close, lequel flux plus ou moins pur (q) est ensuite dilué dans un débit (Q) d'air pur.
   Ce dispositif permet par un mode opératoire approprié, et en faisant varier le rapport (q/Q) de définir le seuil de perception de:
   * l'odeur de base (niveau N maximum)
   * l'odeur du Pebax (niveau à zéro ou à N minimum),
   * l'odeur du mélange de l'enceinte (niveau compris N entre les deux précédents).

   La situation de N par rapport à N maximum et N minimum permet de déterminer l'efficacité du Pebax additivé pour l'usage préfiguré.

### Résultats

On constate une analogie des résultats obtenus par le dispositif expérimental en phase dynamique et de ceux obtenus par le dispositif en phase statique.

Tout d'abord, on peut estimer que le Pebax ne présente pas d'odeur spécifique (du moins son odeur n'est pas perceptible).

En phase dynamique, quel que soit le taux de dilution de l'atmosphère polluée, nous constatons un masquage/désodorisation total, par l'acide undécylénique, des odeurs suivantes:
- odeur de cuisine (émanation de friture)
- odeur de tabac.

Il en est de même en phase statique, avec des modules de Pebax de 50 g additivés à raison de 20% d'acide undécylénique, dans des enceintes de 6 litres.

Lorsqu'on met en situation un cylindre de Pebax de 50 g (additivé à 20% environ d'acide undécylénique) dans un local représentant un volume d'environ 300 m³, on constate une désodorisation de ce volume et un masquage partiel des odeurs au profit de l'odeur de l'acide undécylénique.

## Revendications

**1.-** Composition stable comprenant, en poids par rapport au poids de la composition:
(a) de 90 à 5% en poids d'un polymère polyétheresteramide séquencé de formule: 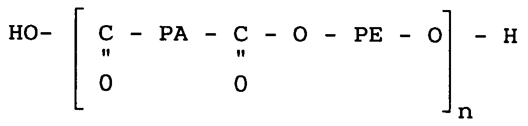 dans laquelle PA représente le segment polyamide et PE représente le segment polyéther, n est un entier représentant la répartition des unités récurrentes; et
(b) 10 à 95% en poids, d'acide undécylénique ou ses dérivés, calculé sur la base du poids en acide undécylénique.

**2.-** Composition selon la revendication 1, comprenant: 50 à 10% de polymère polyétheresteramide séquencé, et 50 à 90% d'acide undécylénique ou ses dérivés.

**3.-** Composition selon la revendication 1 ou 2, dans laquelle le dérivé d'acide undécylénique est un ester d'alkyle en C₁ à C₆.

**4.-** Articles façonnés obtenus directement à partir de la composition selon les revendications 1 à 3.

**5.-** Semelle intérieure de chaussure comprenant une composition selon les revendications 1 à 3.

**6.-** Mélange-maître obtenu à partir de la composition selon les revendications 1 à 3.

**7.-** Procédé de préparation d'une composition selon les revendications 1 à 3, caractérisé en ce que l'acide undécylénique et/ou ses dérivés est incorporé dans un alcool, selon un rapport volumique, calculé par rapport à la forme acide, comprise entre 1/9 et 9/1, et imprégnation de polyétheresteramide avec ce mélange résultant.

**8.-** Procédé de préparation selon la revendication 7, caractérisé en ce que l'alcool est ensuite totalement ou partiellement éliminé.

**9.-** Procédé de préparation selon la revendication 7 ou 8, caractérisé en ce que l'alcool est le méthanol.

**10.-** Procédé de préparation selon l'une quelconque des revendications 7 à 9, caractérisé en ce que ce rapport est compris entre 1/2 et 2/1.
